(19) Europäisches Patentamt
European Patent Office
Office européen des brevets

(11) Veröffentlichungsnummer: **0 501 249 A1**

# (12) EUROPÄISCHE PATENTANMELDUNG

(21) Anmeldenummer: **92102448.5**

(51) Int. Cl.5: **C07D 221/14**, G03G 9/097

(22) Anmeldetag: **14.02.92**

(30) Priorität: **27.02.91 DE 4106122**

(43) Veröffentlichungstag der Anmeldung: **02.09.92 Patentblatt 92/36**

(84) Benannte Vertragsstaaten: **CH DE FR GB LI**

(71) Anmelder: **BAYER AG**

**W-5090 Leverkusen 1 Bayerwerk(DE)**

(72) Erfinder: **Berneth, Horst, Dr.**
**Erfurter Strasse 1**
**W-5090 Leverkusen 1(DE)**
Erfinder: **Harnisch, Horst, Dr.**
**Heinenbusch 4**
**W-5203 Much(DE)**
Erfinder: **Raue, Roderich, Dr.**
**Berta-von-Suttner-Strasse 48**
**W-5090 Leverkusen 1(DE)**
Erfinder: **Hassdenteufel, Jürgen-Rolf, Dr.**
**Mutzer Strasse 114**
**W-5060 Bergisch Gladbach 2(DE)**
Erfinder: **Köcher, Matthias, Dr.**
**Eichen 46**
**W-5603 Overath(DE)**

## (54) Neue Naphthalimide, diese enthaltende Toner und die Verwendung der neuen Naphtalimide als Additive für Toner.

(57) Naphthalimide der Formel (I)

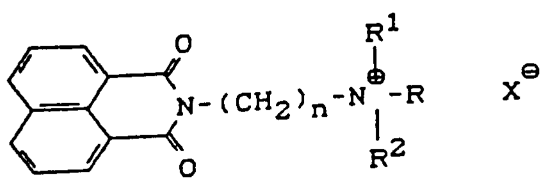

(I),

in der

| | |
|---|---|
| R | für $C_1$- bis $C_{22}$-Alkyl steht, |
| $R^1$ und $R^2$ | unabhängig voneinander jeweils für $C_1$-bis $C_4$-Alkyl stehen oder $R^1$ und $R^2$ gemeinsam mit dem dazwischenliegenden Stickstoffatom einen Pyrroldidin-, Piperidin- oder Morpholinring bilden, |
| n | für 2 oder 3 steht und |
| $X^\ominus$ | für ein Äquivalent eines Benzoats oder Naphthoats, das gegebenenfalls mit Chlor, Brom, Methyl, Methoxy, Hydroxy, Cyano und/oder Hydroxycarbonyl substituiert ist, eines mit Hydroxy, Amino oder Nitro substituierten Benzolsulfonats, eines Naphthalinsulfonats, das gegebenenfalls durch Hydroxy, Amino oder eine Sulfonsäuregruppe substituiert ist oder eines mit Fluor substituierten Anions einer $C_4$- bis $C_{18}$-Alkancarbon-oder Alkansulfonsäure steht. |

Diese Naphthalimide können als Additive zur Ladungs- und Leitfähigkeitskontrolle in Tonern verwendet werden. Sie zeichnen sich durch höhere Aufladung, Langzeitstabilität und Unempfindlichkeit gegen Luftfeuchtigkeit aus.

EP 0 501 249 A1

Die vorliegende Erfindung betrifft Naphthalimide und deren Verwendung als Additive zur Ladungs- und Leitfähigkeitskontrolle in Tonern.

Toner werden bei der Entwicklung elektrostatischer Ladungsbilder in der Elektrofotografie, Elektrografie und Ionografie und magnetostatischer Ladungsbilder in der Magnetografie verwendet. Von diesen Verfahren hat die Elektrofotografie (siehe z.B. US-PS 2 297 691, US-PS 3 666 363 und US-PS 4 071 361) die größte Bedeutung erlangt.

Bei dieser Methode wird auf einem Fotoleiter durch Belichtung des Originals oder Umsetzung einer digitalisierten Bildinformation mit Hilfe eines Lasers, eines LED-Arrays oder einer LCS-Einheit ein virtuelles Bild in Form von elektrischen Ladungen erzeugt. Die Entwicklung dieses Ladungsbildes erfolgt durch Kontakt mit einem Toner. Bei dem Toner handelt es sich im allgemeinen um ein Pulver pigmentierter Teilchen, die eine definierte Partikelgröße und definierte elektrische Eigenschaften haben.

Der das Bild aufbauende Toner wird von der Oberfläche des Fotoleiters auf das Trägermaterial (z.B. Papier oder Folie) übertragen und dort fixiert.

Die Entwicklung des Ladungsbildes auf der Oberfläche des Fotoleiters kann nach dem Einkomponentenverfahren oder nach dem Zweikomponentenverfahren erfolgen.

Beim Zweikomponentenverfahren (siehe z.B. US-PS 2 874 063) werden Tonerteilchen mit einer Größe von 5 bis 30 µm und 80 bis 90 Gew.-% Tonerharz, 5 bis 15 Gew.-% Pigment und geringe Mengen Additive enthaltend, durch mechanische Agitation mit magnetischen Trägerteilchen (z.B. aus Roheisen, beschichtetem Roheisen oder Ferriten) mit einer Größe von 30 bis 200 µm triboelektrisch aufgeladen und bilden so eine Schicht auf der Oberfläche der entgegengesetzt aufgeladenen Tragerteilchen. Die Trägerteilchen werden mit Hilfe eines Magnetfeldes an einer rotierenden Walze (sog. magnetische Bürste) radial ausgerichtet und in permanenten Kontakt mit dem ebenfalls rotierenden Fotoleiter gebracht. Die an den Trägerteilchen haftenden Tonerpartikel werden auf die dem virtuellen Ladungsbild entsprechenden entgegengesetzt aufgeladenen Stellen übertragen.

Beim Einkomponentenverfahren (siehe z.B. US-PS 3 909 258) werden Carrier-Toner-Hybridteilchen eingesetzt. Diese haben eine Größe von 5 bis 30 µm und enthalten 40 bis 65 Gew.-% magnetische Pigmente, 30 bis 60 Gew.-% Tonerharz und geringe Mengen Additive. Der Toner wird elektrisch oder triboelektrisch vorgeladen und durch ein radial orientiertes Magnetfeld an der Oberfläche einer rotierenden Walze in räumliche Nähe des Fotoleiters gebracht. Die Tonerteilchen werden auf die dem virtuellen Ladungsbild entsprechenden geladenen Stellen übertragen.

Bei der Fixierung des auf das Trägermaterial (z.B. Papier oder Folie) übertragenen Toners haben sich in der Praxis die Flashfixierung und die Heißwalzenfixierung durchgesetzt.

Bei der Flashfixierung wird der Toner mittels einer Blitzlichtlampe (Xenonlampe) schnell und kontaktlos auf dem Trägermaterial aufgeheizt, so daß er in die Trägeroberfläche einschmilzt.

Bei der Heißwalzenfixierung wird der Träger mit dem anhaftenden Toner in Kontakt mit einer beheizten Walze gebracht, die den Toner unter Druck und Hitze mit der Trägeroberfläche dauerhaft verbindet.

Die Bildqualität der Reproduktion hängt immer wesentlich von den Eigenschaften des Toners ab, wobei die elektrischen Eigenschaften, insbesondere die triboelektrische Aufladung und die Leitfähigkeit für sämtliche Transferprozesse bedeutsam sind. Eine Beeinflussung der elektrischen Eigenschaften ist durch spezielle Additive (z.B. sogenannte Ladungskontrollsubstanzen) oder durch Variation der Pigmente (z.B. Ruß) möglich.

Für eine gute Bildqualität muß das Ladungsniveau und die Leitfähigkeit des Toners der Polarität und Vorspannung des Fotoleiters, der Transferspannung zum Übertrag auf das Trägermaterial und der jeweils verwendeten Vorrichtung (z.B. Kopierer oder Laserprinter) exakt angepaßt sein. Außerdem muß sichergestellt sein, daß die Ladungen und die Leitfähigkeiten einzelner Tonerpartikel nicht oder nicht wesentlich voneinander abweichen.

Wenn die Ladung zu niedrig oder die Leitfähigkeit zu hoch ist, erfolgt keine ausreichende Übertragung von der magnetischen Bürste auf den Fotoleiter, die Entwicklung wird unspezifisch (was zu Unschärfen führt) und der Transfer auf das Trägermaterial wird unzureichend (Anhäufung von sog. "waste-toner").

Bei zu hoher Ladung sind aufgrund ausgeprägter Haftung des Tonerpulvers an der magnetischen Bürste und am Fotoleiter die Transfervorgänge stark behindert und in ihrer Effektivität nicht ausreichend.

Wenn das Tonerpulver Teilchen mit unterschiedlichem Ladungsniveau enthält werden höher geladene Teilchen bevorzugt übertragen und im noch nicht übertragenen Toner erfolgt eine Anreicherung von Teilchen mit niederer Ladung. Hieraus resultiert eine unzulängliche Performance bei langer Betriebsdauer. Äußerst störend wirken Partikel mit umgekehrter Polarität (sogenannte "wrong-sign-particles").

Für den störungslosen Ablauf elektrofotografischer Prozesse ist es auch erforderlich, daß das Ladungsniveau und die Leitfähigkeit der Tonerpartikel möglichst unabhängig von klimatischen Einflüssen ist, z.B. von der Temperatur und der Luftfeuchtigkeit.

Man versucht Tonern die gewünschten Eigenschaften zu verleihen, indem man ihnen sogenannte Ladungskontrollsubstanzen zusetzt, beispielsweise 1 bis 3 Gew.-% spezielle Ammonium- oder Pyridiniumsalze, Metallkomplexe und/oder spezielle Pigmente (siehe US-PS 38 93 935, 39 44 493, 40 07 293, 40 79 014, 42 98 672, 43 38 390, 43 94 439, 44 93 883 und die DE-OS 36 04 827).

Man kann dabei so verfahren, daß man beispielsweise 5 bis 15 Gew.-% übliche Pigmente, 0,5 bis 3 Gew.-% Ladungskontrollsubstanzen und gegebenenfalls weitere Additive (z.B. 1 bis 3 Gew.-% Polyolefinwachse zur Steigerung der adhäsiven Eigenschaften) in das Harz einknetet, dann fein mahlt, nachfolgend zur Einstellung einer definierten Teilchengröße mehrere Sichtungsschritte vornimmt und abschließend mit hochdisperser Kieselsäure oder ähnlichen Produkten die gewünschten Pulverfließeigenschaften einstellt.

Für die Qualität des so erhaltenen Toners ist die Einheitlichkeit der einzelnen Tonerpartikel von größter Wichtigkeit. Daher werden hohe Anforderungen an die Dispergierbarkeit der Additive, insbesondere der Ladungskontrollsubstanzen gestellt.

Des weiteren dürfen die verwendeten Ladungskontrollsubstanzen keine chemischen Wechselwirkungen mit Geräteteilen (z.B. Fotoleiter oder Fixierwalze) eingehen, die zu irreversiblen Verschmutzungen und Beschädigungen der betroffenen Teile führen würden. Hierbei ist insbesondere im Hinblick auf die beheizten Fixierwalzen eine hohe Thermostabilität der Ladungskontrollsubstanzen gefordert.

Zusätzlich zu diesen vielfältigen Anforderungen an Ladungskontrollsubstanzen müssen diese bei der Herstellung von farbigen Fotokopien und farbigen Bildreproduktionen eine gute Transparenz und möglichst nur eine geringe oder am besten gar keine Eigenfarbe aufweisen.

Die in der DE-OS 36 04 827 beschriebenen Ladungskontrollsubstanzen haben sich als brauchbar erwiesen, jedoch ist ihre Aufladbarkeit, ihre Langzeitstabilität und ihre Unempfindlichkeit gegenüber Luftfeuchtigkeit noch nicht voll befriedigend.

Es wurden nun Naphthalimide der Formel (I) gefunden

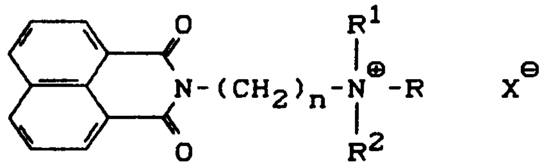

(I),

in der

| | |
|---|---|
| R für | $C_1$- bis $C_{22}$-Alkyl steht, |
| $R^1$ und $R^2$ | unabhängig voneinander jeweils für $C_1$-bis $C_4$-Alkyl stehen oder $R^1$ und $R^2$ gemeinsam mit dem dazwischenliegenden Stickstoffatom einen Pyrroldidin-, Piperidin- oder Morpholinring bilden, |
| n für | 2 oder 3 steht und |
| $X^\ominus$ für | ein Äquivalent eines Benzoats oder Naphthoats, das gegebenenfalls mit Chlor, Brom, Methyl, Methoxy, Hydroxy, Cyano und/oder Hydroxycarbonyl substituiert ist, eines mit Hydroxy, Amino oder Nitro substituierten Benzolsulfonats, eines Naphthalinsulfonats, das gegebenenfalls durch Hydroxy, Amino oder eine Sulfonsäuregruppe substituiert ist oder eines mit Fluor substituierten Anions einer $C_4$- bis $C_{18}$-Alkancarbon- oder Alkansulfonsäure |

steht.

Bei bevorzugten Naphthalimiden der Formel (I) steht R für $C_4$- bis $C_{16}$-Alkyl und/oder $R^1$ und $R^2$ für Methyl oder Ethyl und/oder n für 3 und/oder $X^\ominus$ für ein Äquivalent eines Benzoats, Benzolsulfonats oder Napthalinsulfonats.

Von den mit Fluor substituierten Anionen von $C_4$- bis $C_{18}$-Alkancarbon- und -Alkansulfonsäuren sind solche von Alkansulfonsäuren mit 4 bis 12 C-Atomen bevorzugt.

Von den für Benzoat, Naphthoat, Benzolsulfonat und Naphthalinsulfonat genannten Substituenten können bis zu drei gleiche oder verschiedene gleichzeitig vorhanden sein.

Beispiele für Anionen $X^\ominus$ sind: Benzoat, 2-, 3- und 4-Chlorbenzoat, 2-, 3- und 4-Brombenzoat, Salicylat, 4-Hydroxybenzoat, 3-Hydroxybenzoat, Phthalat, Isophthalat, Terephthalat, 2-, 3- und 4-Nitrobenzoat, 2-, 3- und 4-Cyanobenzoat, 2-, 3- und 4-Methylbenzoat, 2-, 3- und 4-Methoxybenzoat, 2-Hydroxy-3-, 2-Hydroxy-4- und 2-Hydroxy-5-methylbenzoat, 5-Chlorsalicylat, 2,3-, 2,4- und 3,5-Dihydroxybenzoat, die Anionen der 2-Hydroxynaphthalin-1-carbonsäure, 1-Hydroxynaphthalin-2-carbonsäure und 2-Hydroxynaphthalin-3-carbonsäure, 4-Hydroxyphthalat, 2,5- und 4,6-Dihydroxyiso- und -terephthalat, 2-, 3- und 4-Aminobenzoat, 2-Amino-4- und 2-Amino-6-chlorbenzoat, 3-, 4- und 5-Amino-2-hydroxybenzoat, Phenol-2-, -3- und -4-sulfonat, Aminobenzol-2-, -3- und -4-sulfonat, Nitrobenzol-2-, -3- und -4-sulfonat, 4-Nitrophenol-2-sulfonat, Naphthalin-1- und -2-sulfonat, 1-Hydroxynaphthalin-2-, -3-, -4-, -6-, -7- und -8-sulfonat, 2-Hydroxynaphthalin- 1-, -4-, -5- und - 6-sulfonat, 1-Aminonaphthalin-4- und -8-sulfonat, 2-Aminonaphthalin-1-sulfonat, 1-Hydroxynaphthalin-3,6- und -3,8-disulfonat, 1,6- und 1,7-Dihydroxynaphthalin-3-sulfonat, 1- Aminonaphthalin-3,8-disulfonat, 2-Aminonaphthalin-6,8-disulfonat, 6- und 7-Amino-1-hydroxynaphthalin-3- sulfonat, 1-Amino-8-hydro xynaphthalin-2,4-, -3,6- und -4,6-disulfonat, Perfluorbutan-1-sulfonat und Perfluoroctan-1-sulfonat.

Beispiele für den Rest R sind geradkettige, verzweigte und cyclische Alkylreste wie Methyl, Ethyl, 1- und 2-Propyl, 1- und 2-Butyl, 1- und 2-Pentyl, 1- und 2-Hexyl, 1-Octyl, 1-Decyl, 1-Dodecyl, 2-Ethyl-1-hexyl, 1-Tetradecyl, 1-Hexadecyl, 1-Octadecyl, Cyclohexyl und Cyclopentyl.

Die vorliegende Erfindung betrifft weiterhin Toner, die als die positive Ladung verstärkendes Additiv ein oder mehrere Naphthalimide der Formel (I) enthalten. Solche Toner können beispielsweise 0,5 bis 3 Gew.-% dieser Naphthalimide enthalten und im übrigen wie üblich zusammengesetzt ein, Beispielsweise können solche Toner 40 bis 90 Gew.-% Tonerharz, 4 bis 10 Gew.-% Pigmente, z.B. Ruß, 0,5 bis 5 Gew.-% Polyolefine und gegebenenfalls 30 bis 60 Gew.-% magnetische Pigmente enthalten.

Die vorliegende Erfindung betrifft schließlich auch die Verwendung von Naphthalimiden der Formel (I) als die positive Ladung von Tonern verstärkendes Additiv.

In ihrer chemischen Struktur vergleichbare positive Ladungskontrollsubstanzen sind aus der DE-OS 36 04 827 bekannt. Gegenüber den dort beschriebenen Ladungskontrollsubstanzen weisen die Naphthalimide der vor liegenden Erfindung im wesentlichen andere Anionen auf. Neben allen in der DE-OS 36 04 827 genannten Vorteilen weisen die erfindungsgemäßen Naphthalimide überraschenderweise eine wesentlich höhere Aufladung, eine bessere Langzeitstabilität im Gerätetest und eine höhere Unempfindlichkeit gegenüber Luftfeuchtigkeit auf.

Die erfindungsgemäßen Naphthalimide können nach an sich bekannten Methoden, z.B. analog den in den DE-OS'en 35 35 496 und 36 04 827 beschriebenen Verfahren hergestellt werden. Die verschiedensten Anionen $X^{\ominus}$ können beispielsweise durch Anionenaustausch des entsprechenden Bromides hergestellt werden. Dabei kann man $X^{\ominus}$ z.B. in Form der entsprechenden Säure oder der entsprechenden Alkali- oder Ammoniumsalze einsetzen.

## Beispiele

### Beispiel 1

Zu 375 g Naphthalsäureanhydrid in 2355 g Isopropanol und 2,7 g Eisessig wurden 194,4 g 1-Amino-3-dimethylaminopropan bei 80 °C zugetropft. Nach 2 h wurden 63 g Natriumhydrogencarbonat eingetragen und 762,3 g 1-Bromhexadecan bei 70 °C dazugetropft. Nach 15 stündigem Kochen wurde das Lösungsmittel bis zu einer Sumpftemperatur von 105 °C abdestilliert und nach Abkühlen auf 60 °C der Rückstand mit 2370 g Aceton versetzt. Beim Abkühlen kristallisierten 918 g (82,5 % der Theorie) des Bromids aus, das der Formel (I) entsprach mit $R = C_{16}H_{33}$ und $X^{\ominus} = Br^{\ominus}$.

293,8 g dieses Bromids wurden in 2500 ml Wasser suspendiert und 216 g Natriumbenzoat eingetragen. Nach dem Rühren über Nacht wurden 306,4 g (97,5 % der Theorie) hellbeige Kristalle mit einem Schmelzpunkt von 89 bis 91 °C erhalten, die der Formel (I) entsprachen mit $R = C_{16}H_{33}$ und $X^{\ominus}$ = unsubstituiertem Benzoat.

### Beispiel 2

5,9 g des Bromids aus Beispiel 1 wurden in 50 ml Ethanol suspendiert und 1,2 g Propylenoxid (zur Entfernung von $Br^{\ominus}$) und 4,2 g Naphthalin-2-sulfonsäure zugesetzt.

Nach 2 stündigem Rühren wurde abgesaugt und der Rückstand mit Ethanol gewaschen. Es wurden 6,4 g (89 % der Theorie) eines hellbeigen Kristallpulvers erhalten, das der Formel (I) entsprach mit $R = C_{16}H_{33}$ und $X^{\ominus}$ = unsubstituiertes Naphthalin-2-sulfonat. Die Kristalle hatten einen Schmelzpunkt von 195 bis 200 °C.

### Beispiele 3 bis 23

Analog den Beispielen 1 oder 2 wurden folgende Verbindungen hergestellt:

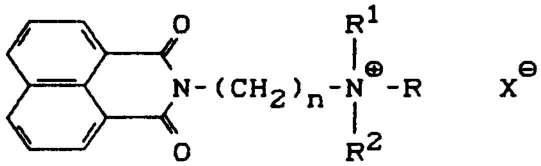

(I)

Wenn nichts anders angegeben ist, ist $R^1 = R^2 =$ Methyl und $n = 3$.

Tabelle 1

| Beispiel Nr. | analog Beispiel | R | $X^{\ominus}$ | Schmelzpunkt (°C) |
|---|---|---|---|---|
| 3 | 1 | $C_{16}H_{33}$ | 2-Hydroxybenzoat | 133 bis 135 |
| 4 | 1 | $C_{16}H_{33}$ | Phthalat | 162 bis 164 |
| 5 | 1 | $C_{16}H_{33}$ | 1-Sulfonato-2-hydroxynaphthalin | 184 bis 187 |
| 6 | 2 | $C_{16}H_{33}$ | 1-Hydroxy-3-sulfonato-6-amino-naphthalin | 158 bis 161 |
| 7 | 1 | $C_4H_9$ | 4-Hydroxy-benzoat | 112 bis 115 |
| 8 | 1 | $C_{22}H_{45}$ | 4-Chlorbenzoat | 68 bis 72 |
| 9 $R^1 = R^2 =$ Ethyl | 1 | $C_{16}H_{33}$ | 1-Hydroxy-4-sulfonato-naphthalin | |
| 10 | 2 | $C_{16}H_{33}$ | 3-Nitro-benzoat | 76 bis 77 |
| 11 | 2 | $C_{16}H_{33}$ | 4-Methoxy-benzoat | |
| 12 | 1 | $C_8H_{17}$ | 2-Hydroxy-3-methyl-benzoat | 74 bis 75 |

Tabelle 1

| Beispiel Nr. | analog Beispiel | R | $X^{\ominus}$ | Schmelzpunkt (°C) |
|---|---|---|---|---|
| 13 | 2 | $C_8H_{17}$ | 2-Amino-benzoat | |
| 14 | 1 | $C_{12}H_{25}$ | 2-Hydroxy-benzol-sulfonat | |
| 15 | 1 | $C_{16}H_{33}$ | 2-Hydroxy-4-amino-benzolsulfonat | 163-165 |
| 16 $R^1$ = $R^2$ = Butyl | 1 | $C_{16}H_{33}$ | 6-Sulfonato-2-hydroxy-naphthalin | |
| 17 | 1 | $C_{16}H_{33}$ | 3-Sulfonato-1,7-dihydroxy-naphthalin | 125-127 |
| 18 | 1 | $C_{16}H_{33}$ | 6-Sulfonato-1-amino-naphthalin | |
| 19 | 2 | $C_{16}H_{33}$ | 6-Sulfonato-2-amino-naphthalin | |
| 20 $N(R^1)(R^2)$ = Morpholin, n = 2 | 1 | $C_4H_9$ | 1-Amino-3-sulfonato-8-hydroxy-6-naphthalin-sulfonsäure | |

Tabelle 1

| Beispiel Nr. | analog Beispiel | R | $X^{\ominus}$ | Schmelzpunkt (°C) |
|---|---|---|---|---|
| 21 | 1 | $C_{14}H_{29}$ | 1,8-Dihydroxy-3-sulfonato-6-naphthalin-sulfonsäure | |
| 22 | 2 | $C_{16}H_{33}$ | Perfluorbutansulfonat | 202-203 |
| 23 | 1 | $C_{16}H_{33}$ | Perfluoroctansulfonat | |
| 24 | 1 | $C_{16}H_{33}$ | 2-Hydroxybenzoat | |

$N(R^1)(R^2)$ = Piperidin

Beispiel 25 (Tonerbeispiel)

90 g Styrol/Butylmethacrylat-Copolymer wurden mit 7 g Ruß und 3 g des nach Beispiel 4 erhaltenen Naphthalimidderivats bei 150 °C verknetet. Nach Zerkleinerung, grober Vormahlung und anschließender

Vermahlung in einer Strahlmühle wurde durch Sichtung ein Toner mit einer Partikelgröße $d_{50}$ von 13 µm erhalten. Die triboelektrische Aüfladung gegenüber einem Reineisencarrier betrug 20,5 µC/g. Sie wurde von der Luftfeuchtigkeit praktisch nicht beeinflußt. Im Langzeit-Gerätetest wurde keine Abnahme der Aufladung festgestellt.

Ähnlich gut verhalten sich uch die Naphthalimidderivate der Beispiele 1-3 und 5-24.

Beipiel 26 (Vergleichsbeispiel)

Die Herstellung eines Toners erfolgte wie in Beispiel 25, jedoch wurde ein Naphthalimid-Derivat eingesetzt, das sich von demjenigen des Beispiels 4 dadurch unterschied, daß es anstelle eines Phthalatanions ein Bromidanion enthielt, wie es aus der DE-OS 36 04 827 bekannt ist.

Der Toner zeigte eine niedrigere Anfangsaufladung von 15 µC/g. Die triboelektrische Aufladung gegenüber einem Reineisencarrier nahm nach 10-stündiger Agitation mit einem Reineisencarrier auf 5 µC/g ab. Im Gerätetest zeigten sich analoge Phänomene.

Beispiel 27 (Vergleichsbeispiel)

Die Herstellung eines Toners erfolgte wie in Beispiel 25, jedoch wurde ein Naphthalimidderivat eingesetzt, das sich von demjenigen des Beispiels 4 dadurch unterschied, daß es anstelle eines Phthalatanions ein Hydrogensulfatanion enthielt, wie es aus der DE-OS 36 04 827 bekannt ist, Der Toner zeigte eine niedrigere Anfangsladung von 14,5 µC/g, die nach 10-stündiger Agitation mit einem Reineisencarrier auf 7 µC/g abnahm. Im Gerätetest zeigten sich analoge Phänomene.

Beispiel 28 bis 32 (Tonerbeispiel)

Analog der Tonerpräparation nach Beispiel 25 wurden die folgenden Toner hergestellt und wie in Beispiel 25 beschrieben getestet:

Tabelle 2

| Beispiel Nr. | Naphthalimid aus Beispiel-Nr. | Aufladung (µC/g) |
|---|---|---|
| 28 | 3 | 19,7 |
| 29 | 5 | 17,9 |
| 30 | 6 | 16,9 |
| 31 | 9 | 18,5 |
| 32 | 7 | 17,8 |

Beispiel 33 (Tonerbeispiel)

90 g Styrol/Butylacrylat-Copolymer wurden mit 7 g Ruß, 3 g Polypropylen und 1,8 g des nach Beispiel 4 erhaltenen Naphthalimidderivats bei 150 °C verknetet. Nach Zerkleinerung, grober Vormahlung und anschließender Vermahlung in einer Strahlmühle wurde durch Sichtung ein Toner mit einer Partikelgröße $d_{50}$ von 13 µm erhalten. Die triboelektrische Aufladung gegenüber einem Ferritcarrier betrug 27,8 µC/g. Sie wurde von der Luftfeuchtigkeit praktisch nicht beeinflußt` Im Langzeit-Gerätetest wurde keine Abnahme der Aufladung festgestellt.

Ähnlich gut verhalten sich auch die Naphthalimidderivate der Beispiele 1-3 und 5-24.

Beispiel 34 (Vergleichsbeispiel)

Die Herstellung eines Toners erfolgte wie in Beispiel 33, jedoch wurde ein Naphthalimid-Derivat eingesetzt, das sich von demjenigen des Beispiels 4 dadurch unterschied, daß es anstelle des Phthalatanions ein Bromidanion enthielt, wie es aus der DE-OS 36 04 827 bekannt ist.

Der Toner zeigte eine niedrigere Anfangsaufladung von 17 µC/g. Die triboelektrische Aufladung gegenüber einem Reineisencarrier nahm nach 10-stündiger Agitation mit einem Ferritcarrier auf 3 µC/g ab, Im Gerätetest zeigten sich analoge Phäomene.

Beispiel 35 (Vergleichsbeipiel)

Die Herstellung eines Toners erfolgte wie in Beispiel 33, jedoch wurde ein Naphthalimidderivat eingesetzt, das sich vom demjenigen des Beispiels 4 dadurch unterschied, daß es anstelle eines Phthalatanions ein Hydrogensulfatanion enthielt, wie es aus der DE-OS 36 04 827 bekannt ist. Der Toner zeigte eine niedrigere Anfangsladung von 11,7 µC/g, die nach 10-stündiger Agitation mit einem Ferritcarrier auf 5 µC/g abnahm. Im Gerätetest zeigten sich analoge Phänomene.

Beispiele 36-40

Analog Beispiel 33 wurden Tonerpräparationen hergestellt und getestet. Ergebnisse siehe Tabelle 3.

Tabelle 3

| Beispiel Nr. | Naphthalimid Nr. | Aufladung (µC/g) |
|---|---|---|
| 36 | 3 | 25,2 |
| 37 | 5 | 23,4 |
| 38 | 6 | 27,3 |
| 39 | 9 | 27,9 |
| 40 | 7 | 33,2 |

## Patentansprüche

**1.** Naphthalimide der Formel (I)

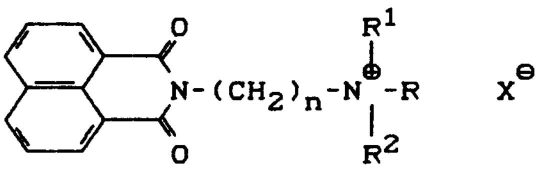

(I),

in der

R für $C_1$- bis $C_{22}$-Alkyl steht,

$R^1$ und $R^2$ unabhängig voneinander jeweils für $C_1$-bis $C_4$-Alkyl stehen oder $R^1$ und $R^2$ gemeinsam mit dem dazwischenliegenden Stickstoffatom einen Pyrroldidin-, Piperidin- oder Morpholinring bilden,

n für 2 oder 3 steht und

$X^{\ominus}$ für ein Äquivalent eines Benzoats oder Naphthoats, das gegebenenfalls mit Chlor, Brom, Methyl, Methoxy, Hydroxy, Cyano und/oder Hydroxycarbonyl substituiert ist, eines mit Hydroxy, Amino oder Nitro substituierten Benzolsulfonats, eines Naphthalinsulfonats, das gegebenenfalls durch Hydroxy, Amino oder eine Sulfonsäuregruppe substituiert ist oder eines mit Fluor substituierten Anions einer $C_4$- bis $C_{18}$-Alkancarbon- oder Alkansulfonsäure

steht.

**2.** Naphthalimide nach Anspruch 1, dadurch gekennzeichnet, daß in Formel (I) R für $C_4$- bis $C_{16}$-Alkyl und/oder $R^1$ und $R^2$ für Methyl oder Ethyl und/oder n für 3 und/oder $X^{\ominus}$ für ein Äquivalent eines Benzoats, Benzolsulfonats oder Naphthalinsulfonats stehen.

**3.** Naphthalimide nach Anspruch 1, dadurch gekennzeichnet, daß in Formel (I) $X^{\ominus}$ für ein Äquivalent einer Alkansulfonsäure mit 4 bis 12 C-Atomen steht,

**4.** Naphthalimide nach Anspruch 1, dadurch gekennzeichnet, daß in Formel (I) von den bei $X^{\ominus}$ = Benzoat,

Naphthoat, Benzolsulfonat und Naphthalinsulfonat genannten Substituenten gleichzeitig bis zu drei gleiche oder verschiedene vorhanden sind.

5. Naphthalimide nach Anspruch 1, dadurch gekennzeichnet, daß in Formel (I) $X^{\ominus}$ für Benzoat, 2-, 3-und 4-Chlorbenzoat, 2-, 3- oder 4- Brombenzoat, Salicylat, 4-Hydroxybenzoat, 3-Hydroxybenzoat, Phthalat, Isophthalat, Terephthalat, 2-, 3- oder 4-Nitrobenzoat, 2-, 3- oder 4-Cyanobenzoat, 2-, 3-oder 4-Methylbenzoat, 2-, 3- oder 4-Methoxybenzoat, 2-Hydroxy-3-, 2-Hydroxy-4- oder 2-Hydroxy-5-methylbenzoat, 5-Chlorsalicylat, 2,3-, 2,4- oder 3,5-Dihydroxybenzoat, die Anionen der 2-Hydroxynaphthalin-1-carbonsäure, 1-Hydroxynaphthalin-2-carbonsäure oder 2-Hydroxynaphthalin-3-carbonsäure, 4-Hydroxyphthalat, 2,5- oder 4,6-Dihydroxyiso- oder -terephthalat, 2-, 3- und 4-Aminobenzoat, 2-Amino4-oder 2-Amino-6-chlorbenzoat, 3-, 4- oder 5-Amino2-hydroxybenzoat, Phenol-2-, -3- oder -4-sulfonat, Aminobenzol-2-, -3- oder -4-sulfonat, Nitrobenzol-2-, -3- oder -4-sulfonat, 4-Nitrophenol-2-sulfonat, Naphthalin-1- und -2-sulfonat, 1-Hydroxynaphthalin-2-, -3-, -4-, -6-, -7- oder -8-sulfonat, 2-Hydroxynaphthalin-1-, -4-, -5- oder -6-sulfonat, 1-Aminonaphthalin-4- oder -8-sulfonat, 2-Aminonaphthalin-1-sulfonat, 1-Hydroxynaphthalin-3,6-oder -3,8-disulfonat, 1,6- oder 1,7-Dihydroxynaphthalin-3-sulfonat, 1-Aminonaphthalin-3,8-disulfonat, 2-Aminonaphthalin-6,8- disulfonat, 6- oder 7-Amino-1-hydroxynaphthalin-3- sulfonat, 1-Amino-8-hydroxynaphthalin-2,4-, -3,6- oder -4,6-disulfonat, Perfluorbutan-1-sulfonat oder Perfluoroctan-1-sulfonat steht.

6. Naphthalimide nach Anspruch 1, dadurch gekennzeichnet, daß in Formel (I) R für Methyl, Ethyl, 1- und 2-Propyl, 1- und 2-Butyl, 1- und 2-Pentyl, 1- und 2-Hexyl, 1-Octyl, 1-Decyl, 1-Dodecyl, 2-Ethyl-1-hexyl, 1-Tetradecyl, 1-Hexadecyl, 1-Octadecyl, Cyclohexyl und Cyclopentyl steht.

7. Toner, dadurch gekennzeichnet, daß sie als die positive Ladung verstärkendes Additiv ein oder mehrere Napthalimide des Anspruchs 1 enthalten.

8. Toner nach Anspruch 7, dadurch gekennzeichnet, daß sie 0,5 bis 3 Gew.-% der Naphthalimide enthalten.

9. Toner nach Ansprüchen 7 und 8, dadurch gekennzeichnet, daß sie außer den Naphthalimiden 40 bis 90 Gew.-% Tonerharz, 4 bis 10 Gew.-% Pigmente, 0,5 bis 5 Gew.-% Polyolefine und gegebenenfalls 30 bis 60 Gew.-% magnetische Pigmente enthalten.

10. Verfahren zur Herstellung von Naphthalimiden des Anspruchs 1, dadurch gekennzeichnet, daß man von Bromid verschiedene Anionen $X^{\ominus}$ durch Anionenaustausch aus dem entsprechenden Bromid herstellt.

Europäisches Patentamt

# EUROPÄISCHER RECHERCHENBERICHT

Nummer der Anmeldung

EP 92 10 2448

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X<br><br>D | EP-A-0 233 544 (BAYER AKTIENGESELLSCHAFT)<br>* Ansprüche *<br>& DE-A-3 604 827 (BAYER AKTIENGESELLSCHAFT)<br>--- | 1-10 | C07D221/14<br>G03G9/097 |
| X | PATENT ABSTRACTS OF JAPAN<br>vol. 13, no. 505 (P-959)(3853) 14. November 1989<br>& JP-A-01 202 760 ( TOMOEGAWA PAPER CO LTD ) 15. August 1989<br>* Zusammenfassung *<br>--- | 1-10 | |
| A | DE-A-3 738 948 (BAYER AKTIENGESELLSCHAFT)<br>* Ansprüche *<br>--- | 1-10 | |
| A | EP-A-0 154 740 (XEROX CORPORATION)<br>* Ansprüche *<br>----- | 1-10 | |

| RECHERCHIERTE SACHGEBIETE (Int. Cl.5 ) |
|---|
| C07D<br>G03G |

Der vorliegende Recherchenbericht wurde für alle Patentansprüche erstellt

| Recherchenort | Abschlußdatum der Recherche | Prüfer |
|---|---|---|
| DEN HAAG | 14 MAI 1992 | HENRY J.C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)